Europäisches Patentamt

European Patent Office

Office européen des brevets

(11) Publication number: **0 382 832 B1**

# EUROPEAN PATENT SPECIFICATION

(45) Date of publication of patent specification: **23.03.94**  (51) Int. Cl.<sup>5</sup>: **G01N 33/543**, G01N 21/64, G01N 21/55

(21) Application number: **89909006.2**

(22) Date of filing: **25.07.89**

(86) International application number:
**PCT/GB89/00844**

(87) International publication number:
**WO 90/01166 (08.02.90 90/04)**

Consolidated with 89307581.2/0353937
(European application No./publication No.) by
decision dated 30.01.92.

(54) **METHOD OF ASSAY FORA LIGAND IN A SAMPLE.**

(30) Priority: **25.07.88 GB 8817710**

(43) Date of publication of application:
**22.08.90 Bulletin 90/34**

(45) Publication of the grant of the patent:
**23.03.94 Bulletin 94/12**

(84) Designated Contracting States:
**AT BE CH DE ES FR GB GR IT LI LU NL SE**

(56) References cited:
EP-A- 0 075 353      EP-A- 0 170 376
EP-A- 0 171 148      EP-A- 0 286 195
EP-A- 0 305 109      EP-A- 0 346 016
WO-A-88/07202        GB-A- 2 174 802
GB-A- 2 185 308      US-A- 4 649 280

OPTICS LETTERS, vol. 4, no. 8, August 1979,
Optical Society of America (US); W.H. WE-
BER et al., pp. 236-238&NUM;

(73) Proprietor: **ARS Holding 89 N.V.**
**6 John B. Gorsiraweg**
**Curaçao(AN)**

(72) Inventor: **ATTRIDGE, John, Worthington**
**135 Howbury Street**
**Bedford MK40 3OT(GB)**
Inventor: **SHANKS, Ian, Alexander**
**Flintwood Cottage**
**Channer Drive**
**Penn Bucks HP10 8HT(GB)**

(74) Representative: **Woodman, Derek et al**
**Frank B. Dehn & Co.**
**European Patent Attorneys**
**Imperial House**
**15-19 Kingsway**
**London WC2B 6UZ (GB)**

EP 0 382 832 B1

OPTICS COMMUNICATIONS, vol. 30, no. 2, August 1979; R.E. BENNER et al., pp. 145-149&NUM;

# EP 0 382 832 B1

**Description**

This invention relates to assay techniques and to means for putting such techniques into effect. In particular it relates to an improved assay technique which provides an enhanced signal to noise ratio and enhanced sensitivity.

The assay techniques with which the present application is concerned are based on the affinity between the species which is to be assayed (hereinafter called "ligand") and a specific binding material for the ligand (hereinafter called "specific binding partner") which is coated on a particular type of surface. Such techniques are well known in the art, particularly in relation to coated optical structures whereby binding of the ligand to the specific binding partner results in a detectable change in the optical characteristics of said optical structure and have been described, for example, in EP-0112721 and EP-0178083. The present invention provides an alternative method of assay with considerable advantages over the conventional assays.

WO-A-88/07202, which forms part of the state of the art according to Article 54(3) EPC, discloses a method of assaying for a ligand which utilises a diffraction grating capable of exhibiting surface plasmon resonance having bound to its surface a specific binding partner for ligand it is desired to detect. The use of fluorophor-labelled material in an embodiment of such an assay is also disclosed.

EP-A-0346016, which also forms part of the state of the art according to Article 54(3) EPC, discloses a sensor for use in biological, biochemical or general chemical testing and in which the phenomenon of long-range surface plasmon resonance is used. The optical structure employed in the sensor has a block of material transparent to the source radiation, a membrane of dielectric material positioned against a surface of said block, a layer of metal applied to the surface of the membrane opposite said block and a suitable layer of sensitive material applied to said layer of metal. The transparent material and the membrane of dielectric material are disclosed as being in the same integral structure only in an example in which the optical structure takes the form of a continuous film which is movable from a supply reel to a take-up reel. This document does not disclose the use of fluorescent or phosphorescent binding compounds in connection with the sensors described.

In its broadest aspect, the invention is concerned with improvements to a method of assaying for a ligand in a sample which involves

a) incubating the sample in contact with a specific binding partner for the ligand it is desired to detect carried on one surface of an optical structure;

b) irradiating another surface of the optical structure at a suitable angle or range of angles to the normal such that resonance and/or total internal reflection of the radiation occurs within the optical structure and/or the layer of specific binding partner; and

c) analysing the reflected and/or generated radiation in order to determine whether, and if desired the extent to which and/or rate at which, the generated radiation and/or optical characteristics of the optical structure are altered by complex formation.

It is to be understood that the term generated radiation as used herein includes fluorescence and phosphorescence. Although the invention is described hereinafter with particular reference to fluorescence, it applies also to phosphorescence.

The optical structure comprises a substrate and optionally one or more layers of material interposed between said substrate and the layer of specific binding partner carried on the surface of said optical structure. Generally the layer of specific binding partner may be either continuous or discontinuous. However, in some embodiments of the invention as hereinafter described and in particular where the specific binding partner is carried directly on the substrate surface of the optical structure without any intervening layers of material therebetween, a continuous layer of specific binding partner may be preferred.

Unexpectedly, the method of the present invention has been found to be of general applicability for increasing the sensitivity of both direct and indirect sensing methods of assay based on the optical properties of certain surfaces. Direct sensing in the context of the present invention involves monitoring the modulation of a signal resulting from a biochemical reaction (e.g. antigen/antibody binding). Indirect sensing involves monitoring a label (e.g. a fluorophore) by a transducer in order to quantify a biochemical reaction.

The technique makes it possible to enhance substantially the intensity of the electric field at the surface of the optical structure thereby enhancing the interaction between the exciting radiation and the ligand/specific binding partner complex, so maximising the response to complex formation at the surface of the optical structure and, in those embodiments employing indirect sensing techniques, significantly reducing the background signal levels.

Thus, where a direct sensing method is used based on the change in refractive index of the surface layer carried on an optical structure upon binding of the ligand under assay, the method of the present

3

invention may be applied to enhance the surface field intensity produced by the incident radiation source and to sharpen the resonance(s) associated with the coupling to modes which propagate in said surface layer.

Thus, in one aspect, the invention provides a method of assaying for a ligand in a sample which method comprises the steps of

(a) incubating the sample in contact with a specific binding partner for the ligand it is desired to detect, the said specific binding partner being carried on one surface of an optical structure, said optical structure comprising a transparent substrate coated with a thin metal layer which is itself coated with a layer of dielectric material of a thickness suitable to support one or more guided modes, the said specific binding partner being carried on the surface of said layer of dielectric material of the optical structure;

(b) irradiating another surface of said optical structure such that the radiation is totally internally reflected and is coupled to a guided mode supported by the layer of dielectric material; and

(c) analysing the reflected radiation in order to determine whether, and if desired the extent to which and/or rate at which, the optical characteristics of the sensor formed by the optical structure and specific binding partner carried thereon are altered by formation of a complex between the ligand the specific binding partner.

In this embodiment of the invention the incident radiation may be coupled to a guided mode which can be supported by an optical structure of a particular geometry. Optical structures of suitable geometry have been previously disclosed in US4649280 in connection with a fluorescent immunoassay. However, it has not hitherto been appreciated that similar techniques could be applied to a direct sensing method of assay. The optical structure comprises a transparent (at least at the wavelength of radiation used) substrate coated with a thin metal layer such as silver or gold, which metal layer is itself coated with a layer of dielectric material such as silica. The layer of dielectric material is of a thickness, for example of the order of a wavelength of the incident radiation used, sufficient to support one or more guided modes but it is particularly preferred to employ a thickness of dielectric material which will support only a single guided TE or TM mode. For example, for a substrate of refractive index 1.52 with a silver layer of 50 nm and an incident radiation wavelength of 543 nm, the thickness of a dielectric layer of silica for a single transverse magnetic (TM) guided mode is from 350 nm to 750 nm. A similar thickness is required to propagate a single guided mode of transverse electric (TE) radiation.

The scope of the invention also extends to a sensor for detecting a ligand in a sample by the aforesaid method, which sensor comprises an optical structure having a substrate coated with a thin layer of metal, which metal layer is itself coated with a layer of dielectric material of a thickness suitable to support one or more guided modes of radiation of wavelength employed when the sensor is in use and which dielectric layer carries a specific binding partner for the ligand it is desired to detect.

The invention also includes a kit for use in the aforesaid method of assay, which comprises

(a) a sensor as defined hereinbefore;

(b) a source of radiation suitable to produce resonant guided mode coupling within the optical structure comprised in said senor;

(c) means for analysing radiation which, in use, is reflected from said sensor.

In an alternative direct sensing embodiment of the invention the incident radiation is coupled to a long-range surface plasmon mode which results from the interaction of surface plasmons on each side of the metal layer.

We therefore provide a method of assaying for a ligand in a sample which method comprises the steps of

(a) incubating the sample in contact with a specific binding partner for the ligand it is desired to detect, the said specific binding partner being carried on one surface of an optical structure, said optical structure being other than in the form of a continuous film and said optical structure comprising a substrate, a layer of metal and interposed therebetween a layer of dielectric material having a refractive index lower than that of said substrate, the said specific binding partner being directly or indirectly adsorbed on or bound to the said metal layer;

(b) irradiating another surface of said optical structure such that the radiation is totally internally reflected and long-range surface plasmon resonance occurs in the optical structure; and

(c) analysing the reflected radiation in order to determine whether, and if desired the extent to which and/or rate at which, the optical characteristics of the sensor formed by the optical structure and specific binding partner carried thereon are altered by formation of a complex between the ligand and the specific binding partner.

In this embodiment the optical structure may, for example, comprise a glass substrate and the layer of metal, such as silver or gold, is displaced from the substrate by a layer of dielectric having a refractive index lower than that of the substrate, for example $MgF_2$. Long-range surface plasmon resonance (LRSPR) is conventionally associated with a geometry in which the refractive indices of the dielectric layers on each side of a metal layer are identical. However, we have found that LRSPR can still be achieved when there is a modest index mis-match and that a range of sensitivities are possible by using layers of differing thicknesses and/or by selecting materials of appropriately mismatched refractive index. Thus, if the dielectric layer thickness is decreased, the metal layer thickness needs to be increased to optimise resonance coupling between the incident radiation and the long-range surface plasmon.

The invention therefore further provides a sensor for detecting a ligand in a sample which comprises an optical structure other than in the form of a continuous film, said optical structure comprising a substrate, a layer of metal and interposed therebetween a layer of dielectric material having a refractive index lower than that of said substrate, and a specific binding partner for the ligand it is desired to detect adsorbed on or bound to (directly or indirectly) the said metal layer, said layers being such that, in use, long range surface plasmon resonance may be propagated therein.

Preferably the metal layer is of silver, 10-50 nm thick, more particularly 15.5 nm thick and the dielectric layer is of $MgF_2$ of 10-2000 nm thick, more particularly 1500 nm thick.

The scope of the invention also extends, to a kit for use in the above method of assay, which comprises

(a) a sensor as defined above for use in the said method;

(b) a source of radiation suitable to produce a long-range surface plasmon resonance within the optical structure comprised in said sensor;

(c) means for analysing radiation which, in use, is reflected from or generated by said sensor.

The sensitivity of direct sensing assays can be conveniently estimated from the resolution of the sensor. The resolution may be defined as the ratio of the angular shift in the resonance peak for a particular change in refractive index of the medium adjacent to the metal surface to the angular half width of the reflected resonance minimum. "Angular half width" as used herein means the angular range between those angles of incidence or reflectance, on either side of the angle of incidence or emergence associated with the resonance reflectance minimum at which the reflectance is at half its minimum value. The greater the resolution the greater is the sensitivity of the sensing system in resolving the changes in resonance due to ligand binding, hence improving the assay sensitivity. In comparable arrangements and for the same change in refractive index, the preferred materials and dimensions described in the first embodiment above give an r value of greater than 5 and those in the second embodiment above give an r value of greater than 12 (with an angular half width of about one sixth of a degree) compared to an r value of 0.47-0.76 and an angular half width of about 1.5 degrees using a bare silver film.

However, the method of the present invention is particularly advantageous when applied to an indirect sensing method of assay such as those techniques based on surface-bound fluorophores. As already described for the direct sensing techniques, the method may be used to enhance the surface field intensity produced by the incident radiation and to sharpen the resonance peaks produced. This in itself produces large improvements in the specificity and sensitivity of fluorescence assays because the bound fluorophores are excited by the evanescent field produced at the outer surface of the optical structure. This minimises excitation of unbound fluorophores and thus reduces background signal. In addition, the surface field intensity is greatly enhanced compared to both direct irradiation and evanescent irradiation via total internal reflection and thus the available energy is greater and the signal obtained from the bound fluorophore very significantly enhanced.

However, still further advantages can be obtained by coupling the emitted fluorescence to the detector via the optical structure and the angular range of the detector can be limited to ensure that substantially only that radiation emitted by the bound fluorophore is detected. Furthermore, by placing the detector outside the plane of irradiation/reflectance, a further decrease in the background signal may be achieved. Filtration of the detected light will then be required only to remove scattered, as opposed to reflected, excitation radiation. Suitable arrangements for the optical detectors to measure the fluorescence emission of bound fluorophores, coupled via total internal reflection or surface plasmon resonance into the optical structure, have been described in, for example, EP-0170376 and the use of evanescent field excitation using total internal reflection has also been disclosed in US4608344, US4447546 and US4558014. However, it has not previously been appreciated that the methods could be generally applicable, in a modified manner, alone or in combination, to provide substantial advantages over the prior art.

In another aspect, the invention provides a method of assaying for a ligand in a sample which method comprises the steps of

(a) incubating the sample in contact with a specific binding partner for the ligand it is desired to detect, the said specific binding partner being carried on one surface of an optical structure and in the presence of a further reagent X being <u>either</u> a fluorescently or phosphorescently labelled ligand analogue specific for the same specific binding partner or a fluorescently or phosphorescently labelled further specific binding partner for the ligand it is desired to detect, said optical structure comprising a substrate and optionally one or more layers of material interposed between said substrate and the said specific binding partner;

(b) irradiating another surface of said optical structure in a plane perpendicular to the plane of the said layers of material and at a suitable angle or range of angles to the normal such that the radiation is totally internally reflected and such that fluorescence or phosphorescence is generated; and

(c) monitoring the generated fluorescence or phosphorescence which emerges from an edge of said optical structure, and analysing said fluorescence or phosphorescence in order to determine whether, and if desired the extent to which and or rate at which, it is altered by complex formation, whereby the axis of the detection optics is substantially at right angles to the plane in which the optical structure is irradiated and reflection therefrom occurs.

For example, in one embodiment of the invention the evanescent field associated with total internal reflection can be used to excite fluorophores within about one micron of the optical structure - sample interface. The optical structure is irradiated with a single reflection in a plane substantially at right angles to the axis of the detection optics. This provides an advantage over the arrangements disclosed in the aforementioned US patents because the detected radiation and the source radiation are in different planes and thus resolution of incident and emitted radiation is simplified. Filtration of the detected light will be required only to remove scattered radiation, although such scattering of radiation emitted due to background solution fluorescence is substantially eliminated using evanescent excitation.

Examples of fluorescent molecules which are suitable for use as labels are rhodamine isothiocyanate, dansyl chloride, FITC and XRITC.

In a further aspect, the invention provides A method of assaying for a ligand in a sample which method comprises the steps of

(a) incubating the sample in contact with a specific binding partner for the ligand it is desired to detect, the said specific binding partner being carried on one surface of an optical structure and in the presence of a further reagent being either a fluorescently or phosphorescently labelled ligand analogue specific for the same specific binding partner or a fluorescently or phosphorescently labelled further specific binding partner for the ligand it is desired to detect, said optical structure comprising a substrate, a layer of metal and optionally a layer of dielectric material interposed between said layer of metal and the layer of specific binding partner carried on the surface of said optical structure, said optical structure being a planar waveguide, prism, optical fibre or slide and not comprising a grating;

(b) irradiating another surface of said optical structure at a suitable angle or range of angles to the normal such that surface plasmon resonance occurs within or at the surface of the optical structure, such that fluorescence or phosphorescence is generated; and

(c) analysing the fluorescence or phosphorescence generated in order to determine whether, and if desired the extent to which and/or rate at which, it is altered by complex formation.

In an embodiment of this aspect of the invention the incident radiation is coupled into a surface plasmon resonance (SPR) mode generated between a thin metal layer, for example of silver or gold, and a dielectric layer which may, for example, be of silica, phosphate glasses or a silane (e.g. glycidoxypropyltrimethoxysilane). Silica can act as a passivating layer to protect the metal from corrosion and to provide a surface on which the specific binding partner can be conveniently immobilised, for example, covalently. However, it will be appreciated that the specific binding partner may be directly adsorbed onto the metal layer to itself form the layer of dielectric material and in this particular embodiment it is preferred that said specific binding partner forms a continuous layer, at least over a discrete region of the optical structure. It has been shown that a surface plasmon's evanescent field intensity is greatly enhanced, compared to that associated with total internal reflection, due to the focussing effect of coupling the incident radiation to a two-dimensional surface wave. The surface field intensity attainable using surface plasmon resonance is wavelength dependent, being greater at longer wavelengths within the optimised optical structure.

Fluorophores within the evanescent field will be excited by a surface plasmon of the appropriate wavelength and enhanced emission will occur. Thus, the general advantages of enhanced surface field intensity and specificity of excitation are attained according to the method of the invention. However, these advantages can again be magnified greatly using a reciprocal optical arrangement whereby the excited fluorophore is able to return to the ground state by coupling its emission to a surface plasmon of the Stoke's shifted wavelength. In this embodiment, enhanced fluorescent emission will occur over a narrow range of angles governed by the surface plasmon dispersion and the fluorophore emission spectrum (see, for example, Benner et al, Optics Communications 30, 145-149 (1979)). The subsequent radiation of the surface plasmon energy can then be detected by an optical arrangement similar to that described in EP-0170376 mentioned hereinbefore. As with evanescent irradiation alone, unbound solution fluorophore (i.e. fluorophore which is at a distance from the surface which is substantially greater than the wavelength of the incident radiation being used) can only be excited by the scattering of incident radiation but in view of the narrow angle of fluorescence emission of fluorophores within the evanescent field and the surface plasmon resonance properties of the metal film, any such solution signal will be still further attenuated by the metal film and hence the background signal further reduced. The coupling probability of the excited fluorophore to the surface plasmons of the metal can be controlled by suitably spacing the specific binding partner layer away from the metal layer (see, for example, Weber and Eagan, Optics Letters 4, 236 (1979)).

In a still further embodiment of the invention the evanescent field associated with long range surface plasmon resonance is employed to excite surface-bound fluorophores.

Accordingly, in a still further aspect of the invention, there is provided a method of assaying for a ligand in a sample which method comprises the steps of

(a) incubating the sample in contact with a specific binding partner for the ligand it is desired to detect, the said specific binding partner being carried on one surface of an optical structure and in the presence of a further reagent being either a fluorescently or phosphorescently labelled ligand analogue specific for the same specific binding partner or a fluorescently or phosphorescently labelled further specific binding partner for the ligand it is desired to detect, said optical structure comprising a substrate, a layer of metal and interposed therebetween a layer of dielectric material having a refractive index lower than that of said substrate, the said specific binding partner being directly or indirectly adsorbed on or bound to the said metal layer;

(b) irradiating another surface of said optical structure at a suitable angle or range of angles to the normal such that long-range surface plasmon resonance occurs and such that fluorescence or phosphorescence is generated; and

(c) analysing the fluorescence or phosphorescence generated in order to determine whether, and if desired the extent to which and/or rate at which, it is altered by complex formation.

A sensor as described hereinbefore in which LRSPR may be propagated is suitable for use in such assays. In all respects the advantages of using LRSPR are the same as those previously discussed for surface plasmon resonance except that the surface field enhancement is greater (x10) than for surface plasmon resonance and the emission angles are narrower which is of particular advantage where the emitted light is itself coupled via LRSPR into the optical structure.

The methods of the present invention have become realistically attainable due to a number of modifications of the instrumentation required, both for irradiating the optical structure and for analysing the reflected, transmitted and/or propagated radiation. Thus, the present invention further provides apparatus suitable for use in a method of assay hereinbefore described which comprises (a)a sensor, the said sensor comprising a specific binding partner for a ligand it is desired to assay carried on the surface of an optical structure comprising a substrate and one or more layers of material interposed between said substrate and said specific binding partner; (b)a collimated source of radiation which is capable of being arranged such that, in use, the radiation enters said optical structure at an angle suitable to produce total internal reflection and optionally resonance (said resonance being surface plasmon resonance, long-range surface plasmon resonance or resonant guided mode coupling) within said optical structure; and (c) means for in use analysing reflected or generated radiation.

The radiation may be collimated, for example, to within one or two degrees and may, in use, be introduced into an optical structure positioned within said apparatus, for example, through an edge of the substrate of the optical structure or via a prism or a grating coupler. Ideally the source radiation is polarised, preferably transverse magnetic polarised, but an unpolarised radiation source may also be used.

Where the method of assay involves coupling the fluorescence of surface bound fluorophores into the optical structure, for example by total internal reflection, SPR or LRSPR, the sensitivity of the method may be further enhanced using apparatus wherein the angular range of view of the detector means is restricted, for example to about 3°, to correspond to the coupled fluorescence emission angles. A theoretical analysis of this effect is given in EP-0170376.

It is particularly preferred to apply the method of the invention to an immunoassay and in particular to use a specifically reactive sample collecting and testing device similar to that described in EP-0171148, together with the method of optical analysis disclosed in EP-0170376. Thus, the present invention provides a specifically-reactive sample collecting and testing device possessing a cavity or cavities each having a dimension small enough to enable sample liquid to be drawn into the cavity by capillary action, and wherein at least one part of a wall of said cavity comprises a sensor for detecting a ligand in a sample, said sensor being a sensor of the invention as described herein. In this particular embodiment of the invention the optical structure comprises a planar waveguide.

In a preferred embodiment of such a device, the wall of the capillary cavity which is remote from the wall comprising a sensor carries in dry releasable form a fluorescently or phosphorescently labelled ligand analogue or a further specific binding partner.

However, it will be appreciated that the optical structure used in the method of assay according to the invention is not limited to planar waveguides and includes within its scope other optical structures such as gratings, (apart from the specific exception indicated hereinbefore) prisms, optical fibers and slides, such as gratings, prisms, optical fibres and slides, provided that a suitable geometry can be chosen for introducing the incident radiation other than via the sample and at a suitable angle or range of angles such that resonance and/or total internal reflection can occur.

In a preferred embodiment of the invention the ligand is an antigen and the specific binding partner comprises an antibody to the said antigen. However, the invention is not to be taken as limited to assays of antibodies or antigens. Examples of ligands which may be assayed by the method of the invention are given in Table 1 below, together with an indication of a suitable specific binding partner in each instance.

Table 1

| Ligand | Specific Binding Partner |
|---|---|
| antigen | specific antibody |
| antibody | antigen |
| hormone | hormone receptor |
| hormone receptor | hormone |
| polynucleotide strand | complementary polynucleotide strand |
| avidin | biotin |
| biotin | avidin |
| protein A | immunoglobulin |
| immunoglobulin | protein A |
| enzyme | enzyme cofactor (substrate) or inhibitor |
| enzyme cofactor (substrate) or inhibitor | enzyme |
| lectins | specific carbohydrate |
| specific carbohydrate of lectins | lectins |

The method of the invention has very broad applicability but in particular may be used to assay: hormones, including peptide hormones (e.g. thyroid stimulating hormone (TSH), luteinising hormone (LH), human chorionic gonadotrophin (hCG) , follicle stimulating hormone (FSH), insulin and prolactin) or non-peptide hormones (e.g. steroid hormones such as cortisol, estradiol, progesterone and testosterone, or thyroid hormones such as thyroxine (T4) and triiodothyronine), proteins (e.g. carcinoembryonic antigen (CEA) and alphafetoprotein (AFP)), drugs (e.g. digoxin), sugars, toxins, vitamins, viruses such as influenza, parainfluenza, adeno-, hepatitis, respiratory and AIDS viruses, or microorganisms.

It will be understood that the term "antibody" used herein includes within its scope:

(a) any of the various classes or sub-classes of immunoglobulin, e.g. IgG, IgA, IgM, or IgE derived from any of the animals conventionally used, e.g. sheep, rabbits, goats or mice,

(b) monoclonal antibodies,

(c) intact molecules or "fragments" of antibodies, monoclonal or polyclonal, the fragments being those which contain the binding region of the antibody, i.e. fragments devoid of the Fc portion (e.g. Fab, Fab', $F(ab')_2$) or the so-called "half-molecule" fragments obtained by reductive cleavage of the disulphide bonds connecting the heavy chain components in the intact antibody.

The method of preparation of fragments of antibodies is well known in the art and will not be described herein.

The term "antigen" as used herein will be understood to include both permanently antigenic species (for example, proteins, bacteria, bacterial fragments, cells, cell fragments and viruses) and haptens which may be rendered antigenic under suitable conditions.

The following non-limiting Examples illustrate particular aspects of the invention.

Example 1 Comparison of resonance excitation geometries

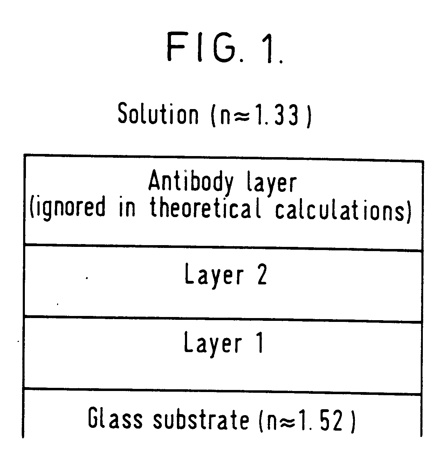

Table 2 below shows a comparison of the results theoretically obtainable using an optical structure as depicted schematically in Figure 1. The excitation wavelength for all mechanisms was 543 nm and the fluorophore was rhodamine B with a peak emission at 570 nm and a half width of 35 nm (from 555 nm to 590 nm).

Example 2 Total internal reflection

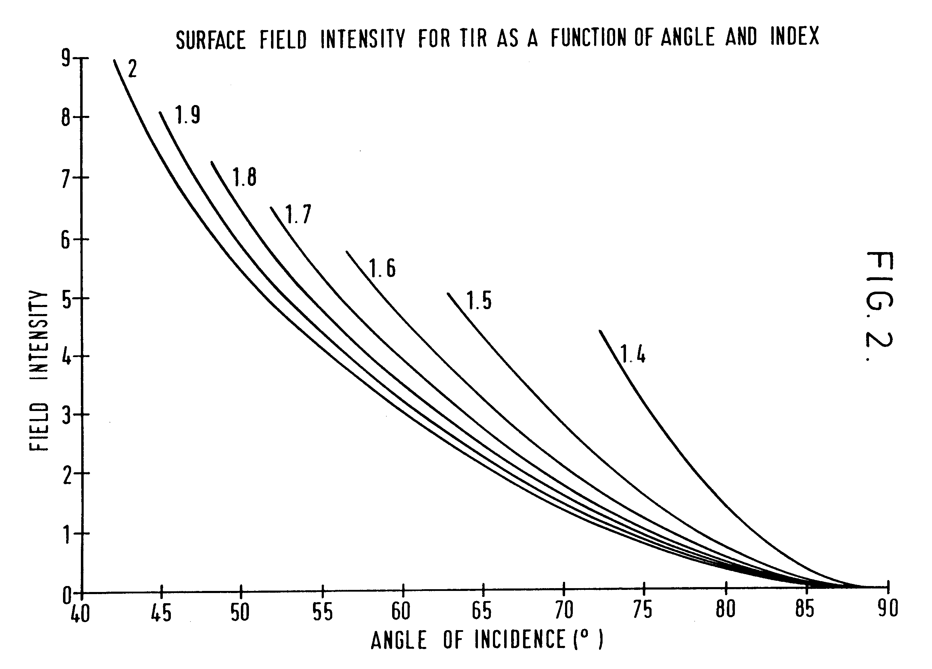

a) Figure 2 shows the surface field intensity plotted as a function of angle for a number of glass indices in water, with a wavelength of 543 nm (green HeNe laser). For a glass slide with a refractive index of 1.52 the field intensity is 2.5 times that of the incident radiation at 70°.

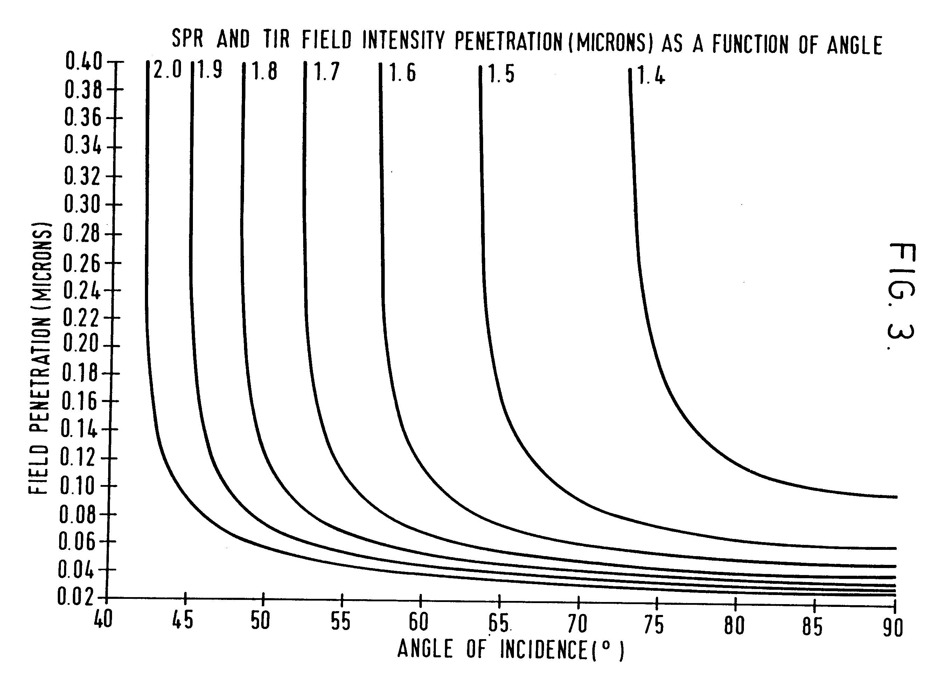

b) Figure 3 shows the field intensity penetration plotted as a function of angle. Away from the critical angle this is a slowly varying function of the angle of incidence. For the example given in (a) above the field penetration is about 90 nm at 70°. It is not desirable to work too close to the critical angle because the penetration is too great and poor surface discrimination would result. Thus, a balance between field intensity and penetration depth must be struck.

Example 3 Surface plasmon resonance

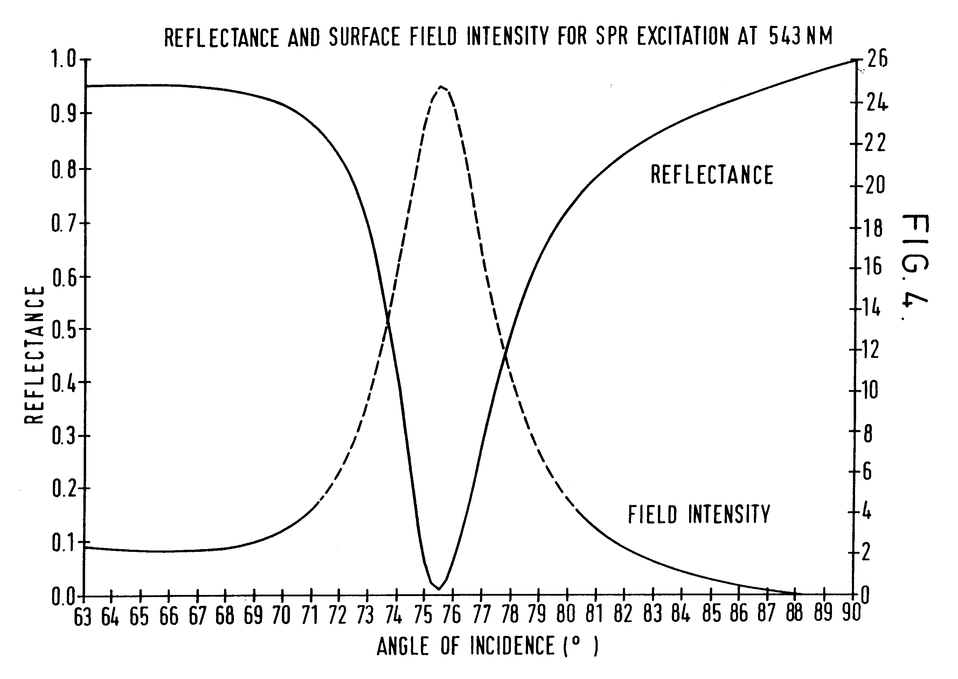

Figure 4 shows the reflectance and surface field intensity of a 50 nm silver film in water irradiated at 543 nm. A twenty-five-fold enhancement of the field intensity results when compared with the intensity of the incident radiation.

Table 2

| Mechanism | Layer 1 | Layer 2 | Excitation angle | Emission peak angle | Emission half width |
|---|---|---|---|---|---|
| SPR | Ag 50 nm | $SiO_2$ 10 nm | 72.80° | 70.72° | 1.92° |
| LRSPR | $MgF_2$ 1000 nm | Ag 26 nm | 65.67° | 65.31° | 0.39° |
| LRSPR | $MgF_2$ 500 nm | Ag 40 nm | 67.61° | 66.80° | 0.84° |

Example 4 Comparison of SPR and total internal reflection

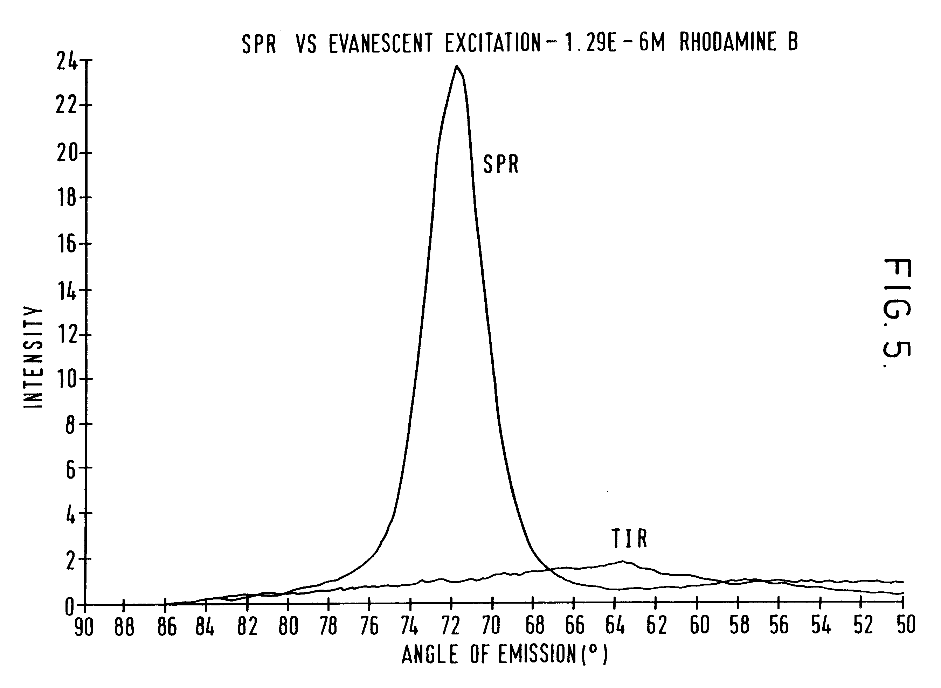

Figure 5 shows a comparison of the excitation of a rhodamine B solution by surface plasmon resonance and total internal reflection. The emission intensity enhancement and narrow emission range for the SPR geometry can be clearly seen. The experimental details follow.

(i) Fabrication of an Optical Structure

Glass slides measuring 25 mm x 75 mm were cleaned ultrasonically in a solution of detergent. Using vacuum deposition, chromium was deposited onto one half of one surface of each slide through a mask, to a thickness of 1 nm. In the same way, silver was deposited onto the chromium layer to a thickness of 54 nm. The mask was removed and a 10 nm coating of silica was deposited onto the whole of said surface by similar means, to ensure that both halves of the surface of each slide had the same physico-chemical properties. The slides were washed with ultra-pure water. Each slide was scribed and cut into three pieces (such that each piece was half-silvered). Each piece was used to make a capillary cell using another piece of glass of similar size and double-sided adhesive tape.

(ii) Experimental Procedure

A sample solution was made up using an appropriate concentration of a suitable protein labelled with rhodamine B. The experimental set up was as described in section (iii) of Example 5, below.

Example 5 Assay for human chorionic gonadotrophin (hCG) using an indirect surface plasmon resonance technique

(i) Fabrication of an Optical Structure

Glass slides measuring 25mm x 75mm were cleaned ultrasonically in a solution of detergent. Using vacuum deposition, aluminium was deposited onto one half of one surface of each slide through a mask, to a thickness of 1nm. In the same way silver was deposited onto the aluminium layer to a thickness of 54nm. The mask was removed and a 10nm coating of silica was deposited onto the whole of said surface by similar means, to ensure that both halves of the surface of each slide had the same surface chemistry. The slides were suspended 5mm above a pool of GOPS (glycidoxypropyltrimethoxysilane) for 2 hours at 20°C in order to silanize the silica surface, following which the slides were baked at 60°C for one hour. One 75ul drop of a 20ug/ml solution of α12/17 anti-hCG antibody in HEPES buffer was placed on each half of each slide, and the slides were left to dry for two hours. The slides were washed with ultra-pure water and then a solution of sucrose with tris(hydroxymethyl)aminomethane and sodium aside was deposited on the surface of the slide by means of spinning. Each slide was scribed and cut into three pieces (such that each piece was half-silvered). Each piece was used to make a capillary cell using another piece of glass of similar size and double-sided adhesive tape.

(ii) Assay Methodology

A sandwich-type assay was performed using premixed solutions in horse serum of hCG and XRITC-labelled antibody immobilised in the capillary cell. The concentration of XRITC-labelled antibody used was 2.5 ug/ml. Sample solutions were taken up by the capillary cells prepared as in section (i) and allowed to incubate for fifteen minutes before a reading was taken.

(iii) Experimental set-up

The filled capillary cell under study was coupled to a hemicylindrical lens using a fluid of suitable refractive index. Light from a green helium-neon laser was then directed at the slide (i.e. the plate of the cell which carried the immobilised antibody) through the planar wall of the lens at an angle suitable for surface plasmon resonance to occur. Fluorescence emission was monitored by rotating a photomultiplier tube in a plane perpendicular to the plane of the incident/reflected light. The light reaching the PMT detector passed through a 610 nm bandpass filter to remove any scattered excitation light. A slit was placed behind the filter to give an angular resolution of 1°. Two lenses focussed the light passing through the slit onto the detector. A comparison of the fluorescence arising from surface plasmon resonance with that arising from total internal reflection was made by sliding the cell on the prism so that the silvered and unsilvered halves of the cell were interrogated in turn.

11

## Results

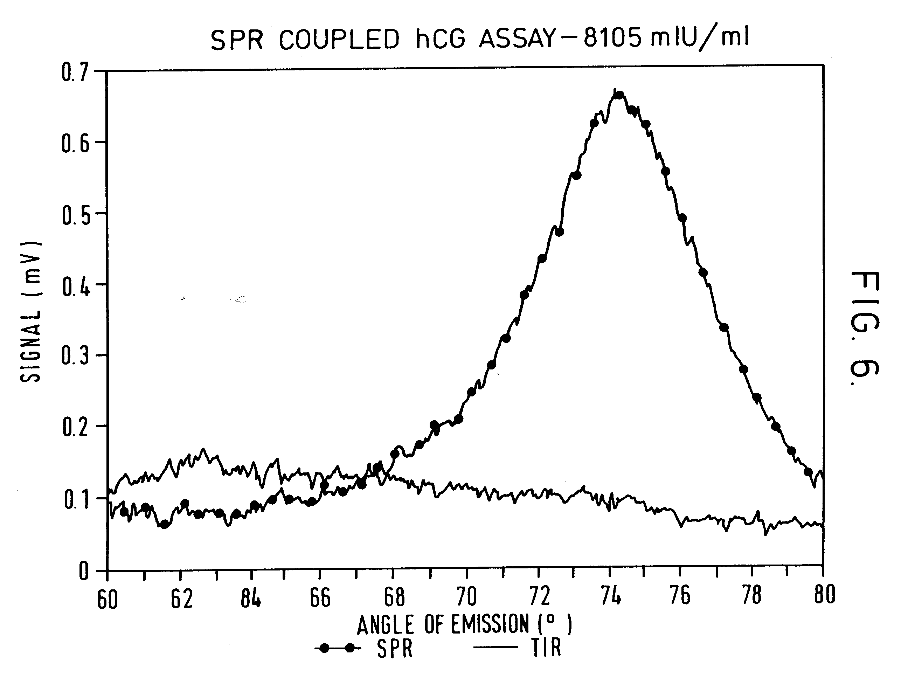

Figure 6 shows a comparison of the signals obtained over the angular emission range 60-80° from a capillary cell containing 8105 mIU/ml hCG in serum, as obtained by SPR and TIR excitation. Particularly noticeable is the strong peak in signal around 74° for SPR excitation. The integrated signal between 70° and 78° is 5.2 times greater using SPR excitation than using TIR excitation. Optimisation of the metal layer and improved protein immobilisation techniques should enable greater enhancements to be achieved.

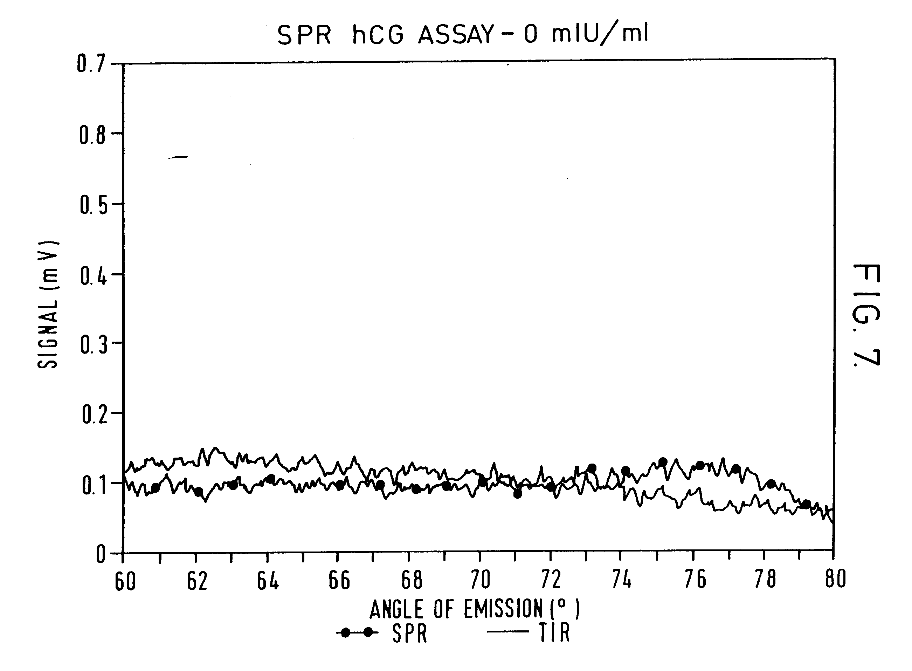

Figure 7 shows signals over the same angular range, but this time from a cell filled with serum containing no hCG.

The emission resulting from SPR excitation shows a slight peak at 76° due to non-specific binding of the XRITC-labelled antibody.

## Example 6 - Demonstration of the sensitivity of a direct guided mode sensor to changes in refractive index.

### (i) Fabrication of the optical structure

Glass microscope slides were cleaned ultrasonically in a detergent solution and extensively rinsed with ultrapure water. Using vacuum deposition techniques, a layer of aluminium, 1nm thick, was deposited onto the surface of the glass followed by a film of silver, 54nm thick. The silver surface was then coated with a glass film by spin coating a silica solgel (HT Products Inc., USA) onto the device at 300 rpm. The optical structures were baked overnight at 60°C. A capillary cell was fabricated from the optical structure and another piece of glass of the same dimensions using double sided adhesive tape.

### (ii) Experimental set-up

The filled capillary cell under study was optically coupled to a right angled crown glass prism using an appropriate fluid. The cell was illuminated through the glass substrate and onto the silver film with a TM polarised HeNe Laser, the reflected light intensity being measured with a photodiode device. The prism was rotated through a range of angles during the measurement. The cell was filled with ultrapure water (refractive index 1.3316) and the position of the reflectance minimum noted. Then ultrapure water was replaced by a 5% solution of sucrose (refractive index of 1.3382) and then by a 10% solution of sucrose (refractive index of 1.3450), the position of the minimum in reflected light intensity being measured in the presence of the sucrose solutions.

### (iii) Results

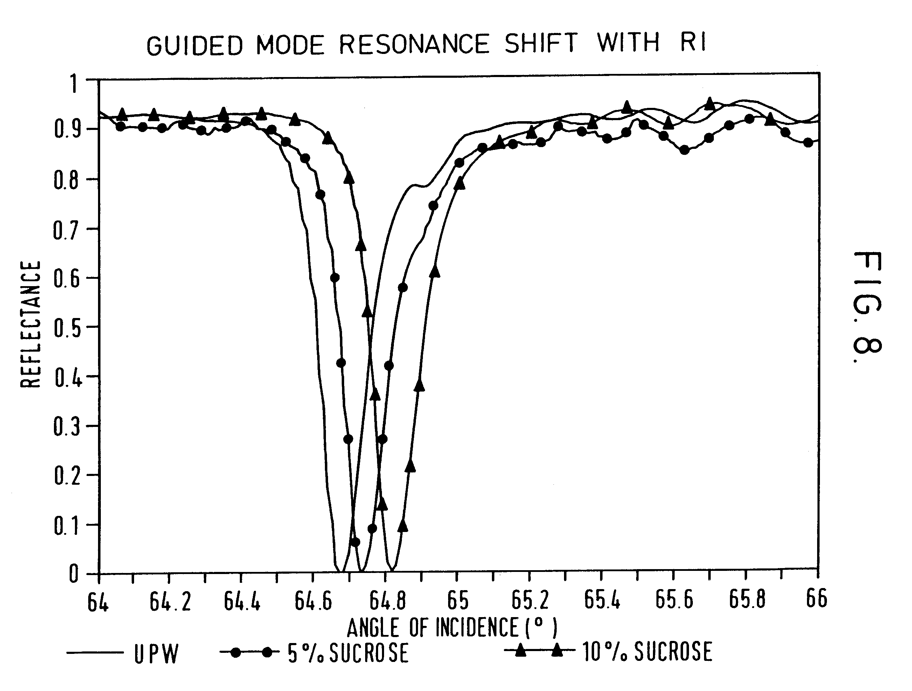

Figure 8 shows the positions of the minima in reflected light intensity of the guided mode light in the optical structure. As the refractive index of the medium in contact with the optical structure increases there is a shift in the angle at which the minimum occurs, demonstrating that the device is sensitive to refractive index changes.

The binding of a ligand (e.g. an antigen) to an appropriate biological molecule (e.g. an antibody) immobilised on the device surface will result in a refractive index change. This will allow the guided mode sensor to be used as a direct optical immunosensor.

## Claims

1. A method of assaying for a ligand in a sample which method comprises the steps of
   (a) incubating the sample in contact with a specific binding partner for the ligand it is desired to detect, the said specific binding partner being carried on one surface of an optical structure and in the presence of a further reagent being either a fluorescently or phosphorescently labelled ligand analogue specific for the same specific binding partner or a fluorescently or phosphorescently labelled further specific binding partner for the ligand it is desired to detect, said optical structure comprising a substrate, a layer of metal and interposed therebetween a layer of dielectric material having a refractive index lower than that of said substrate, the said specific binding partner being directly or indirectly adsorbed on or bound to the said metal layer;
   (b) irradiating another surface of said optical structure at a suitable angle or range of angles to the normal such that long-range surface plasmon resonance occurs and such that fluorescence or phosphorescence is generated; and

12

(c) analysing the fluorescence or phosphorescence generated in order to determine whether, and if desired the extent to which and/or rate at which, it is altered by complex formation.

2. A method of assaying for a ligand in a sample which method comprises the steps of

(a) incubating the sample in contact with a specific binding partner for the ligand it is desired to detect, the said specific binding partner being carried on one surface of an optical structure, said optical structure being other than in the form of a continuous film and said optical structure comprising a substrate, a layer of metal and interposed therebetween a layer of dielectric material having a refractive index lower than that of said substrate, the said specific binding partner being directly or indirectly adsorbed on or bound to the said metal layer;

(b) irradiating another surface of said optical structure such that the radiation is totally internally reflected and long-range surface plasmon resonance occurs in the optical structure; and

(c) analysing the reflected radiation in order to determine whether, and if desired the extent to which and/or rate at which, the optical characteristics of the sensor formed by the optical structure and specific binding partner carried thereon are altered by formation of a complex between the ligand and the specific binding partner.

3. A method of assaying for a ligand in a sample which method comprises the steps of

(a) incubating the sample in contact with a specific binding partner for the ligand it is desired to detect, the said specific binding partner being carried on one surface of an optical structure, said optical structure comprising a transparent substrate coated with a thin metal layer which is itself coated with a layer of dielectric material of a thickness suitable to support one or more guided modes, the said specific binding partner being carried on the surface of said layer of dielectric material of the optical structure;

(b) irradiating another surface of said optical structure such that the radiation is totally internally reflected and is coupled to a guided mode supported by the layer of dielectric material; and

(c) analysing the reflected radiation in order to determine whether, and if desired the extent to which and/or rate at which, the optical characteristics of the sensor formed by the optical structure and specific binding partner carried thereon are altered by formation of a complex between the ligand the specific binding partner.

4. A method of assaying for a ligand in a sample which method comprises the steps of

(a) incubating the sample in contact with a specific binding partner for the ligand it is desired to detect, the said specific binding partner being carried on one surface of an optical structure and in the presence of a further reagent being <u>either</u> a fluorescently or phosphorescently labelled ligand analogue specific for the same specific binding partner <u>or</u> a fluorescently or phosphorescently labelled further specific binding partner for the ligand it is desired to detect, said optical structure comprising a substrate and optionally one or more layers of material interposed between said substrate and the said specific binding partner;

(b) irradiating another surface of said optical structure in a plane perpendicular to the plane of the said layers of material and at a suitable angle or range of angles to the normal such that the radiation is totally internally reflected and such that fluorescence or phosphorescence is generated; and

(c) monitoring the generated fluorescence or phosphorescence which emerges from an edge of said optical structure, and analysing said fluorescence or phosphorescence in order to determine whether, and if desired the extent to which and or rate at which, it is altered by complex formation, whereby the axis of the detection optics is substantially at right angles to the plane in which the optical structure is irradiated and reflection therefrom occurs.

5. A method of assaying for a ligand in a sample which method comprises the steps of

(a) incubating the sample in contact with a specific binding partner for the ligand it is desired to detect, the said specific binding partner being carried on one surface of an optical structure and in the presence of a further reagent being <u>either</u> a fluorescently or phosphorescently labelled ligand analogue specific for the same specific binding partner <u>or</u> a fluorescently or phosphorescently labelled further specific binding partner for the ligand it is desired to detect, said optical structure comprising a substrate, a layer of metal and optionally a layer of dielectric material interposed between said layer of metal and the layer of specific binding partner carried on the surface of said optical structure, said optical structure being a planar waveguide, prism, optical fibre or slide and not comprising a grating;

(b) irradiating another surface of said optical structure at a suitable angle or range of angles to the normal such that surface plasmon resonance occurs within or at the surface of the optical structure, such that fluorescence or phosphorescence is generated; and

(c) analysing the fluorescence or phosphorescence generated in order to determine whether, and if desired the extent to which and/or rate at which, it is altered by complex formation.

6. A sensor for detecting a ligand in a sample by the method of claim 1 or claim 2 which comprises an optical structure other than in the form of a continuous film, said optical structure comprising a substrate, a layer of metal and interposed therebetween a layer of dielectric material having a refractive index lower than that of said substrate, and a specific binding partner for the ligand it is desired to detect adsorbed on or bound to (directly or indirectly) the said metal layer, said layers being such that, in use, long range surface plasmon resonance may be propagated therein.

7. A sensor as claimed in claim 6 wherein the layer of metal is of silver and is between 10 and 50 nm thick, and the layer of dielectric material is of magnesium fluoride and is between 10 and 2000 nm thick.

8. A sensor as claimed in claim 7 wherein the silver layer is 15.5 nm thick and the magnesium fluoride layer is 1500 nm thick.

9. A sensor for detecting a ligand in a sample by the method of claim 3 which comprises an optical structure having a substrate coated with a thin layer of metal, which metal layer is itself coated with a layer of dielectric material of a thickness suitable to support one or more guided modes of radiation of wavelength employed when the sensor is in use and which dielectric layer carries a specific binding partner for the ligand it is desired to detect.

10. A sensor as claimed in claim 9 wherein the layer of metal is of silver or gold and is about 50 nm thick and the layer of dielectric material is of silica.

11. A specifically reactive sample-collecting and testing device possessing a cavity or cavities each having a dimension small enough to enable sample liquid to be drawn into the cavity by capillary action, and wherein at least one part of a wall of said cavity comprises a sensor as claimed in any one of claims 6 to 10.

12. A device as claimed in claim 11 wherein the wall of the capillary cavity which is remote from the wall comprising a sensor carries in dry releasable form a fluorescently or phosphorescently labelled ligand analogue or further specific binding partner.

13. A kit for use in a method of assay as claimed in claim 1 or claim 2 which comprises
(a) a sensor as claimed in any one of claims 6 to 8;
(b) a source of radiation suitable to produce a long-range surface plasmon resonance within the optical structure comprised in said sensor;
(c) means for analysing radiation which, in use, is reflected from or generated by said sensor.

14. A kit for use in a method of assay as claimed in claim 3 which comprises
(a) a sensor as claimed in claim 9 or claim 10;
(b) a source of radiation suitable to produce resonant guided mode coupling within the optical structure comprised in said sensor;
(c) means for analysing radiation which, in use, is reflected from said sensor.

**Patentansprüche**

1. Assayverfahren auf einen Liganden in einer Probe, umfassend die Stufen
(a) Inkubieren der Probe in Kontakt mit einem spezifischen Bindungspartner für den nachzuweisenden Liganden, wobei der spezifische Bindungspartner auf einer Oberfläche einer optischen Struktur aufgebracht ist, und in Gegenwart eines weiteren Reagenzes, das entweder ein fluoreszent oder phosphoreszent markiertes Ligandenanalogon mit Spezifität für den gleichen spezifischen Bindungspartner oder ein fluoreszent oder phosphoreszent markierter weiterer spezifischer Bindungspartner für den nachzuweisenden Liganden ist, wobei die optische Struktur ein Substrat, eine Metallschicht und, dazwischen angebracht, eine Schicht aus einem dielektrischen Material mit einem niedrigeren Brechungsindex als das Substrat umfaßt, wobei der spezifische Bindungspartner direkt oder indirekt an die Metallschicht adsorbiert oder gebunden ist;

(b) Bestrahlen einer anderen Oberfläche der optischen Struktur in einem geeigneten Winkel oder Winkelbereich zu der Normalen, so daß eine Oberflächen-Plasmon-Resonanz über einen weiten Bereich eintritt, und Fluoreszenz oder Phosphoreszenz erzeugt wird; und

(c) Analyse der erzeugten Fluoreszenz oder Phosphoreszenz, um zu bestimmen, ob, und gegebenenfalls in welchem Ausmaß und/oder mit welcher Geschwindigkeit diese durch die Komplexbildung verändert wird.

2. Assayverfahren auf einen Liganden in einer Probe, umfassend die Stufen

(a) Inkubieren der Probe in Kontakt mit einem spezifischen Bindungspartner für den nachzuweisenden Liganden, wobei der spezifische Bindungspartner auf einer Oberfläche einer optischen Struktur aufgebracht ist, wobei die optische Struktur anders als in Form eines kontinuierlichen Films ist, und die optische Struktur ein Substrat, eine Metallschicht und, dazwischen angebracht, eine Schicht aus einem dielektrischen Material mit einem niedrigeren Brechungsindex als das Substrat umfaßt, wobei der spezifische Bindungspartner direkt oder indirekt an die Metallschicht adsorbiert oder gebunden ist;

(b) Bestrahlen einer anderen Oberfläche der optischen Struktur, so daß die Strahlung total intern reflektiert wird und eine Oberflächen-Plasmon-Resonanz über einen weiten Bereich in der optischen Struktur eintritt; und

(c) Analyse der reflektierten Strahlung, um zu bestimmen, ob, und gegebenenfalls in welchem Ausmaß und/oder mit welcher Geschwindigkeit die optischen Eigenschaften des durch die optische Struktur und den darauf angebrachten spezifischen Bindungspartner gebildeten Sensors durch die Bildung eines Komplexes zwischen dem Liganden und dem spezifischen Bindungspartner verändert werden.

3. Assayverfahren auf einen Liganden in einer Probe, umfassend die Stufen

(a) Inkubieren der Probe in Kontakt mit einem spezifischen Bindungspartner für den nachzuweisenden Liganden, wobei der spezifische Bindungspartner auf einer Oberfläche einer optischen Struktur aufgebracht ist, wobei die optische Struktur ein transparentes Substrat, das mit einer dünnen Metallschicht beschichtet ist, die selbst mit einer Schicht aus einem dielektrischen Material mit einer Dicke, die geeignet ist, eine oder mehrere geführte Schwingungsarten zu unterstützen, beschichtet ist, umfaßt, wobei der spezifische Bindungspartner auf der Oberfläche der Schicht aus dielektrischem Material der optischen Struktur aufgebracht ist,

(b) Bestrahlen einer anderen Oberfläche der optischen Struktur, so daß die Strahlung total intern reflektiert wird und an eine geführte Schwingungsart, die von der Schicht aus einem dielektrischen Material unterstützt wird, gekoppelt wird; und

(c) Analyse der reflektierten Strahlung, um zu bestimmen, ob, und gegebenenfalls in welchem Ausmaß und/oder mit welcher Geschwindigkeit die optischen Eigenschaften des durch die optische Struktur und den darauf angebrachten spezifischen Bindungspartner gebildeten Sensors durch die Bildung eines Komplexes zwischen dem Liganden und dem spezifischen Bindungspartner verändert werden.

4. Assayverfahren auf einen Liganden in einer Probe, umfassend die Stufen

(a) Inkubieren der Probe in Kontakt mit einem spezifischen Bindungspartner für den nachzuweisenden Liganden, wobei der spezifische Bindungspartner auf einer Oberfläche einer optischen Struktur aufgebracht ist, und in Gegenwart eines weiteren Reagenzes, das entweder ein fluoreszent oder phosphoreszent markiertes Ligandenanalogon mit Spezifität für den gleichen spezifischen Bindungspartner oder ein fluoreszent oder phosphoreszent markierter weiterer spezifischer Bindungspartner für den nachzuweisenden Liganden ist, wobei die optische Struktur ein Substrat und gegebenenfalls eine oder mehrere Materialschichten, die zwischen dem Substrat und dem spezifischen Bindungspartner angebracht sind, umfaßt;

(b) Bestrahlen einer anderen Oberfläche der optischen Struktur in einer Ebene senkrecht zu der Ebene der Materialschichten und in einem geeigneten Winkel oder Winkelbereich zu der Normalen, so daß die Strahlung total intern reflektiert wird, und Fluoreszenz oder Phosphoreszenz erzeugt wird;

und

(c) Überwachen der erzeugten Fluoreszenz oder Phosphoreszenz, die aus einer Kante der optischen Struktur austritt, und Analyse der Fluoreszenz oder Phosphoreszenz, um zu bestimmen, ob, und gegebenenfalls in welchem Ausmaß und/oder mit welcher Geschwindigkeit diese durch die Komplexbildung verändert wird, wobei die Achse der Detektionsoptik im wesentlichen rechtwinklig zu der Ebene, in der die optische Struktur bestrahlt wird und aus der die Reflexion austritt, steht.

5. Assayverfahren auf einen Liganden in einer Probe, umfassend die Stufen

(a) Inkubieren der Probe in Kontakt mit einem spezifischen Bindungspartner für den nachzuweisenden Liganden, wobei der spezifische Bindungspartner auf einer Oberfläche einer optischen Struktur aufgebracht ist, und in Gegenwart eines weiteren Reagenzes, das entweder ein fluoreszent oder phosphoreszent markiertes Ligandenanalogon mit Spezifität für den gleichen spezifischen Bindungspartner oder ein fluoreszent oder phosphoreszent markierter weiterer spezifischer Bindungspartner für den nachzuweisenden Liganden ist, wobei die optische Struktur ein Substrat, eine Metallschicht und gegebenenfalls eine Schicht aus einem dielektrischen Material, die zwischen der Metallschicht und der auf der Oberfläche der optischen Struktur aufgebrachten Schicht mit dem spezifischen Bindungspartner, angebracht ist, umfaßt, wobei die optische Struktur ein planarer Hohlleiter, ein Prisma, eine optische Faser oder ein Objektträger, ausgenommen ein Gitter, ist;

(b) Bestrahlen einer anderen Oberfläche der optischen Struktur in einem geeigneten Winkel oder Winkelbereich zu der Normalen, so daß Oberflächen-Plasmon-Resonanz innerhalb oder an der Oberfläche der optischen Struktur eintritt, so daß Fluoreszenz oder Phosphoreszenz erzeugt wird; und

(c) Analyse der erzeugten Fluoreszenz oder Phosphoreszenz, um zu bestimmen, ob, und gegebenenfalls in welchem Ausmaß und/oder mit welcher Geschwindigkeit diese durch die Komplexbildung verändert wird.

6. Sensor zum Nachweis eines Liganden in einer Probe mit dem Verfahren nach Anspruch 1 oder 2, umfassend eine optische Struktur, die anders als in Form eines kontinuierlichen Films ist, wobei die optische Struktur ein Substrat, eine Metallschicht und, dazwischen angebracht, eine Schicht aus einem dielektrischen Material mit einem niedrigeren Brechungsindex als das Substrat, und einen (direkt oder indirekt) an die Metallschicht adsorbierten oder gebundenen spezifischen Bindungspartner für den nachzuweisenden Liganden umfaßt, wobei die Schichten so sind, daß bei Verwendung die Oberflächen-Plasmon-Resonanz über einen weiten Bereich darin fortgepflanzt werden kann.

7. Sensor nach Anspruch 6, worin die Metallschicht aus Silber ist und eine Dicke zwischen 10 und 50 nm besitzt, und die Schicht aus dem dielektrischen Material aus Magnesiumfluorid ist und eine Dicke zwischen 10 und 2000 nm besitzt.

8. Sensor nach Anspruch 7, worin die Silberschicht 15,5 nm dick ist, und die Magnesiumfluoridschicht 1500 nm dick ist.

9. Sensor zum Nachweis eines Liganden in einer Probe mit dem Verfahren nach Anspruch 3, umfassend eine optische Struktur mit einem Substrat, das mit einer dünnen Metallschicht beschichtet ist, wobei die Metallschicht selbst mit einer Schicht aus einem dielektrischen Material, das eine Dicke besitzt, die geeignet ist, eine oder mehrere geführte Schwingungsarten der Strahlung der verwendeten Wellenlängen bei Verwendung des Sensors zu unterstützen, beschichtet ist, und wobei die dielektrische Schicht einen spezifischen Bindungspartner für den nachzuweisenden Liganden trägt.

10. Sensor nach Anspruch 9, worin die Metallschicht aus Silber oder Gold ist und etwa 50 nm dick ist, und die Schicht aus dem dielektrischen Material aus Kieselsäure ist.

11. Spezifisch reaktive Probensammel- und -testvorrichtung mit einer Kavität oder Kavitäten, die jeweils klein genug dimensioniert sind, daß eine Probenflüssigkeit durch die Kapillarwirkung in die Kavität gezogen werden kann, und worin mindestens ein Teil einer Wand der Kavität einen Sensor nach einem der Ansprüche 6 bis 10 umfaßt.

12. Vorrichtung nach Anspruch 11, worin die Wand der Kapillarkavität, die von der den Sensor umfassenden Wand entfernt ist, in trockener, freisetzbarer Form ein fluoreszent oder phosphoreszent markiertes

16

EP 0 382 832 B1

Ligandenanalogon oder einen weiteren spezifischen Bindungspartner trägt.

**13.** Kit zur Verwendung bei einem Assayverfahren nach Anspruch 1 oder 2, umfassend

(a) einen Sensor nach einem der Ansprüche 6 bis 8;

(b) eine Strahlungsquelle, die geeignet ist, eine Oberflächen-Plasmon-Resonanz über einen weiten Bereich in der in dem Sensor enthaltenen optischen Struktur zu erzeugen;

(c) Mittel zur Analyse der Strahlung, die bei Verwendung von dem Sensor reflektiert oder erzeugt wird.

**14.** Kit zur Verwendung bei einem Assayverfahren nach Anspruch 3, umfassend

(a) einen Sensor nach Anspruch 9 oder 10;

(b) eine Strahlungsquelle, die geeignet ist, eine resonanzaktive geführte Schwingungskopplung innerhalb der in dem Sensor enthaltenen optischen Struktur zu erzeugen;

(c) Mittel zur Analyse der Strahlung, die bei Verwendung von dem Sensor reflektiert wird.

**Revendications**

**1.** Méthode d'analyse pour un ligand dans un échantillon, laquelle méthode comprend les étapes de

(a) incubation de l'échantillon en contact avec un partenaire de liaison spécifique pour le ligand qu'il est désiré de détecter, ledit partenaire de liaison spécifique étant porté sur une surface d'une structure optique et en la présence d'un réactif de plus étant, soit un analogue du ligand, marqué de manière fluorescente ou phosphorescente, spécifique pour le même partenaire de liaison spécifique, soit un partenaire de liaison spécifique en plus, marqué de manière fluorescente ou phosphorescente, pour le ligand qu'il est désiré de détecter, ladite structure optique comprenant un substrat, une couche de métal, et interposée là entre une couche de matière diélectrique ayant un indice réfringent plus faible que celui dudit substrat, ledit partenaire de liaison spécifique étant directement ou indirectement adsorbé sur ou lié à ladite couche de métal;

(b) irradiation d'une autre surface de ladite structure optique à un angle convenable ou une échelle d'angles à la normale, telle qu'une résonance de plasmon en surface à longue-distance survienne, et telle qu'une fluorescence ou phosphorescence soit générée; et

(c) analyse de la fluorescence ou de la phosphorescence générée afin de déterminer si, et si désiré le degré auquel et/ou la vitesse à laquelle, elle est altérée par formation de complexe.

**2.** Méthode d'analyse pour un ligand dans un échantillon, laquelle méthode comprend les étapes de

(a) incubation de l'échantillon en contact avec un partenaire de liaison spécifique pour le ligand qu'il est désiré de détecter, ledit partenaire de liaison spécifique étant porté sur une surface d'une structure optique, ladite structure optique étant autre que sous la forme d'un film continu, et ladite structure optique comprenant un substrat, une couche de métal, et interposée là entre une couche d'une matière diélectrique ayant un indice réfringent plus faible que celui dudit substrat, ledit partenaire de liaison spécifique étant directement ou indirectement adsorbé sur ou lié à la dite couche de métal;

(b) irradiation d'une autre surface de ladite structure optique telle que la radiation soit totalement réfléchie intérieurement et qu'une résonance de plasmon en surface à longue-distance survienne dans la structure optique; et

(c) analyse de la radiation réfléchie afin de déterminer si, et si désiré le degré auquel et/ou la vitesse à laquelle, les caractéristiques optiques du capteur, formé par la structure optique et un partenaire de liaison spécifique porté là-dessus, sont altérées par formation d'un complexe entre le ligand et le partenaire de liaison spécifique.

**3.** Méthode d'analyse pour un ligand dans un échantillon, laquelle méthode comprend les étapes de

(a) incubation de l'échantillon en contact avec un partenaire de liaison spécifique pour le ligand qu'il est désiré de détecter, ledit partenaire de liaison spécifique étant porté sur une surface d'une structure optique, ladite structure optique comprenant un substrat transparent enduit avec une fine couche de métal, laquelle est elle-même enduite avec une couche de matière diélectrique d'une épaisseur convenable pour supporter un ou plusieurs modes guidés, ledit partenaire de liaison spécifique étant porté sur la surface de ladite couche de matière diélectrique de la structure optique;

(b) irradiation d'une autre surface de ladite structure optique telle que la radiation soit totalement réfléchie intérieurement, et soit couplée à un mode guidé supporté par la couche de matière

17

EP 0 382 832 B1

diélectrique; et

(c) analyse de la radiation réfléchie afin de déterminer si, et si désiré le degré auquel et/ou la vitesse à laquelle, les caractéristiques optiques du capteur, formé par la structure optique et un partenaire de liaison spécifique porté là-dessus, sont altérées par formation d'un complexe entre le ligand et le partenaire de liaison spécifique.

4. Méthode d'analyse pour un ligand dans un échantillon, laquelle méthode comprend les étapes de

(a) incubation de l'échantillon en contact avec un partenaire de liaison spécifique pour le ligand qu'il est désiré de détecter, ledit partenaire de liaison spécifique étant porté sur une surface d'une structure optique et en la présence d'un réactif de plus étant, soit un analogue de ligand, marqué de manière fluorescente ou phosphorescente, spécifique pour le même partenaire de liaison spécifique, soit un partenaire de liaison spécifique en plus, marqué de manière fluorescente ou phosphorescente, pour le ligand qu'il est désiré de détecter, ladite structure optique comprenant un substrat et éventuellement une ou plusieurs couches de matière interposées entre ledit substrat et ledit partenaire de liaison spécifique;

(b) irradiation d'une autre surface de ladite structure optique dans un plan perpendiculaire au plan des dites couches de matière et à un angle convenable ou une échelle d'angles à la normale, telle que la radiation soit totalement réfléchie intérieurement et telle qu'une fluorescence ou phosphorescence soit générée; et

(c) surveillance de la fluorescence ou phosphorescence générée qui émerge à partir d'un côté de ladite structure optique, et analyse de ladite fluorescence ou phosphorescence afin de déterminer si, et si désiré le degré auquel et ou la vitesse à laquelle, elle est altérée par formation de complexe, par laquelle l'axe des optiques de détection est substantiellement à angles droits par rapport au plan dans lequel la structure optique est irradiée, et d'où la réflexion survient.

5. Méthode d'analyse pour un ligand dans un échantillon, laquelle méthode comprend les étapes de

(a) incubation de l'échantillon en contact avec un partenaire de liaison spécifique pour le ligand qu'il est désiré de détecter, ledit partenaire de liaison spécifique étant porté sur une surface d'une structure optique et en la présence d'un réactif de plus étant, soit un analogue de ligand, marqué de manière fluorescente ou phosphorescente, spécifique pour le même partenaire de liaison spécifique, soit un partenaire de liaison spécifique en plus, marqué de manière fluorescente ou phosphorescente, pour le ligand qu'il est désiré de détecter, ladite structure optique comprenant un substrat, une couche de métal, et éventuellement une couche de matière diélectrique interposée entre ladite couche de métal et la couche de partenaire de liaison spécifique portée sur la surface de ladite structure optique, ladite structure optique étant un guide d'onde plan, un prisme, une fibre optique ou une lamelle et ne comprenant pas un réseau;

(b) irradiation d'une autre surface de ladite structure optique à un angle convenable ou une échelle d'angles à la normale, telle qu'une résonance de plasmon en surface survienne à l'intérieur ou à la surface de la structure optique, telle qu'une fluorescence ou phosphorescence soit générée; et

(c) analyse de ladite fluorescence ou phosphorescence générée afin de déterminer si, et' si désiré le degré auquel et/ou la vitesse à laquelle, elle est altérée par formation de complexe.

6. Capteur pour détecter un ligand dans un échantillon par la méthode de la revendication 1 ou de la revendication 2, qui comprend une structure optique autre que sous la forme d'un film continu, ladite structure optique comprenant un substrat, une couche de métal, et interposée là entre une couche de matière diélectrique ayant un indice réfringent plus faible que celui dudit substrat, et un partenaire de liaison spécifique pour le ligand, qu'il est désiré de détecter, adsorbé sur ou lié à (directement ou indirectement) ladite couche de métal, lesdites couches étant telles que, en usage, une résonance de plasmon en surface à longue distance peut être propagée là-dedans.

7. Capteur tel que revendiqué dans la revendication 6 dans lequel la couche de métal est d'argent et est entre 10 et 50 nm d'épaisseur, et la couche de matière diélectrique est de fluorure de magnésium et est entre 10 et 2000 nm d'épaisseur.

8. Capteur tel que revendiqué dans la revendication 7 dans lequel la couche d'argent est de 15.5 nm d'épaisseur, et la couche de fluorure de magnésium est de 1500 nm d'épaisseur.

18

**9.** Capteur pour détecter un ligand dans un échantillon par la méthode de revendication 3, qui comprend une structure optique ayant un substrat enduit avec une fine couche de métal, laquelle couche de métal est elle-même enduite avec une couche de matière diélectrique d'une épaisseur convenable pour supporter un ou plusieurs modes guidés de radiation de longueur d'onde employée quand le capteur est en usage, et laquelle couche diélectrique porte un partenaire de liaison spécifique pour le ligand qu'il est désiré de détecter.

**10.** Capteur tel que revendiqué dans la revendication 9 dans lequel la couche de métal est d'argent ou d'or et est d'environ 50 nm d'épaisseur et la couche de matière diélectrique est de silice.

**11.** Dispositif collecteur d'échantillon spécifiquement réactif, et testeur possédant une cavité ou des cavités, chacune ayant une dimension suffisamment petite pour permettre à un échantillon liquide d'être tiré dans la cavité par action capillaire, et dans laquelle au moins une partie d'un mur de ladite cavité comprend un capteur tel que revendiqué dans l'une quelconque des revendications 6 à 10.

**12.** Dispositif tel que revendiqué dans la revendication 11 dans lequel le mur de la cavité capillaire, qui est éloigné du mur comprenant un capteur, porte sous forme libérable sèche un analogue de ligand marqué de manière fluorescente ou phosphorescente, ou un partenaire de liaison spécifique en plus.

**13.** Kit pour utiliser dans une méthode d'analyse telle que revendiquée dans la revendication 1 ou la revendication 2 qui comprend
(a) un capteur tel que revendiqué dans une quelconque des revendications 6 à 8;
(b) une source de radiation convenable pour produire une résonance de plasmon en surface à longue distance à l'intérieur de la structure optique comprise dans ledit capteur;
(c) un moyen pour analyser une radiation qui, en usage, est refléchie à partir de ou générée par ledit capteur.

**14.** Kit pour utiliser dans une méthode d'analyse telle que revendiquée dans la revendication 3 qui comprend
(a) un capteur tel que revendiqué dans la revendication 9 ou la revendication 10;
(b) une source de radiation convenable pour produire un couplage de mode guidé résonant à l'intérieur de la structure optique comprise dans ledit capteur;
(c) un moyen pour analyser une radiation qui, en usage, est réfléchie à partir dudit capteur.

# FIG. 1.

Solution (n≈1.33)

| |
|---|
| Antibody layer (ignored in theoretical calculations) |
| Layer 2 |
| Layer 1 |
| Glass substrate (n≈1.52) |

SURFACE FIELD INTENSITY FOR TIR AS A FUNCTION OF ANGLE AND INDEX

FIG. 2.

FIELD INTENSITY

ANGLE OF INCIDENCE (° )

EP 0 382 832 B1

SPR AND TIR FIELD INTENSITY PENETRATION (MICRONS) AS A FUNCTION OF ANGLE

FIG. 3.

EP 0 382 832 B1

REFLECTANCE AND SURFACE FIELD INTENSITY FOR SPR EXCITATION AT 543 NM

REFLECTANCE

FIELD INTENSITY

ANGLE OF INCIDENCE (° )

FIG. 4.

EP 0 382 832 B1

# FIG. 5.

SPR VS EVANESCENT EXCITATION – 1.29E – 6M RHODAMINE B

SPR COUPLED hCG ASSAY — 8105 mIU/ml

FIG. 6.

EP 0 382 832 B1

# FIG. 7.

SPR hCG ASSAY - 0 mIU/ml

SIGNAL (mV)

ANGLE OF EMISSION (°)

●—● SPR ——— TIR

# FIG. 8.

GUIDED MODE RESONANCE SHIFT WITH RI